# EUROPEAN PATENT APPLICATION

(11) **EP 4 044 192 A1**
(43) Date of publication of application: **17.08.2022**
(21) Application number: 20887313.3
(22) Date of filing: 28.10.2020
(51) Int. Cl.: G16H 20/10

(54) **METHOD, PROGRAM, DEVICE, AND SYSTEM FOR ASSISTING DETERMINATION OF AT LEAST ONE OF DOSAGE FORM AND DOSAGE OF DRUG**

(30) Priority: 15.11.2019 JP 2019207045
(71) Applicant: Cardio Intelligence Inc., Tokyo, 106-0044 (JP)
(72) Inventor: TAMURA Yuichi, Tokyo 106-0044 (JP); HATANO Kaoru, Tokyo 106-0044 (JP)
(74) Representative: Plougmann Vingtoft a/s
(86) International application number: PCT/JP2020/040466
(87) International publication number: WO 2021/095532

(57) **Abstract**

The present invention enables a health care professional to identify more suitably for a patient at least one of the dosage form and dosage of a drug. A method according to the present invention is used to determine at least one of the dosage form and dosage of a drug which can be administered to a patient, the method including the following steps carried out by a processor: a step for acquiring electrocardiogram data showing an electrocardiogram of the patient to whom a drug has been administered according to a dosage and a dosage form and who is going about daily life; and a step for determining, on the basis of the electrocardiogram data, whether waveform anomalies which can be evaluated from the electrocardiogram may be occurring in the patient, and outputting, on the basis of the result of the determination, information pertaining to whether it is possible to change at least one of the dosage and the dosage form.

## Description

### TECHNICAL FIELD

The present disclosure relates to a method, a program, an apparatus, and a system for supporting determination of at least one of an administration method of drugs or a dosage of drugs.

### BACKGROUND OF THE INVENTION

The administration method indicating a method of administering a drug to a patient, and the dosage indicating an amount of a drug to be administered to a patient are defined for drugs. Patent Document 1 discloses a system for accepting an input of a body weight of a patient, determining a dosage of a drug on the basis of the input body weight, and injecting the determined dosage of the drug into the body of the patient.

### PRIOR ART

### PATENT DOCUMENT

[Patent Document 1] Japanese Unexamined Patent Application Publication No. 2016-064309

### SUMMARY OF INVENTION

### PROBLEMS TO BE SOLVED BY THE INVENTION

Since drugs have side effects, the administration method and dosage common to all people are determined in advance to ensure safety. However, a degree of side effects that may occur due to an administration of a drug is variable within individuals depending on a patient's physical constitution. Therefore, a drug may have an administration method and a dosage that are more suitable for a patient than the predetermined administration method and dosage. The system described in Patent Document 1 does not consider a patient's physical constitution other than his/her body weight because it simply administers a dosage of a drug simply proportional to a body weight. Therefore, there may be (i) an administration method suitable for an individual patient that differs from an administration method determined in advance for that drug and (ii) a dosage suitable for the individual patient that is greater or less than the dosage determined in advance for that drug. In addition, in clinical trials for determining administration methods and dosages of new drugs, there is a possibility that an administration method and a dosage suitable for a patient, serving as a subject participant, can be found by considering the patient's physical constitution.

The present disclosure has been made in view of these points, and its object is to enable a medical worker to specify at least one of an administration method or a dosage of drugs more suitable for a patient.

### MEANS FOR SOLVING THE PROBLEMS

A method according to a first aspect of the present disclosure is a method for determining at least one of an administration method or a dosage of a drug that can be administered to a patient, the method executed by a processor and including steps of: acquiring electrocardiogram data indicating an electrocardiogram occurring in everyday life of a patient who is given the drug with the administration method and the dosage; determining, on the basis of the electrocardiogram data, whether a waveform abnormality that can be evaluated with an electrocardiogram may have occurred in the patient; and outputting information about advisability of modifying at least one of the administration method or the dosage on the basis of a result of the determining.

The method may further include re-acquiring, after at least one of the administration method or the dosage has been modified, electrocardiogram data of the patient who is given the drug with the modified administration method and dosage; determining whether there is the possibility, on the basis of the re-acquired electrocardiogram data; and re-outputting information about the advisability of modifying at least one of the administration method or the dosage on the basis of a result of the determining.

The acquiring step may sequentially acquire the electrocardiogram data from an electrocardiograph worn by the patient in his/her daily life.

The outputting step may determine, as the waveform abnormality, whether there is a possibility of occurrence of a waveform indicating that arrhythmia is present or may occur.

The outputting step may determine, as the waveform abnormality, whether there is a possibility of occurrence of a waveform indicating that an adverse event that can be evaluated with an electrocardiogram is present.

The outputting step may determine whether there is a possibility of occurrence of the waveform abnormality by detecting at least one of QT prolongation, atrial fibrillation, ventricular tachycardia, supraventricular extrasystole, premature ventricular contraction, atrial flutter, or atrial tachycardia in the electrocardiogram data.

The outputting step may determine whether there is a possibility of occurrence of the waveform abnormality by comparing the electrocardiogram data and normal electrocardiogram data indicating an electrocardiogram of the patient in normal times.

The outputting step may transmit information about the advisability of modifying at least one of the administration method or the dosage to an information terminal associated with a medical worker who examines the patient.

The re-outputting step may output information representing a risk of increasing the dosage on the basis of (i) the electrocardiogram data of the patient who is given the drug at the dosage and (ii) the electrocardiogram data of the patient who is given the drug at the modified dosage.

The obtaining step may obtain the electrocardiogram data of the patient who is given the drug for therapeutic purposes or for purposes of clinical trials.

A program according to a second aspect of the present disclosure is a program used for determining at least one of an administration method or a dosage of a drug that can be administered to a patient, the program causing a processor to execute steps of: acquiring electrocardiogram data indicating an electrocardiogram occurring in everyday life of a patient who is given the drug with the administration method and the dosage; determining, on the basis of the electrocardiogram data, whether a waveform abnormality that can be evaluated with an electrocardiogram may have occurred in the patient; and outputting information about advisability of modifying at least one of the administration method or the dosage on the basis of a result of the determining.

An apparatus according to a third aspect of the present disclosure is an apparatus used for determining at least one of an administration method and a dosage of a drug that can be administered to a patient, the apparatus including: an acquisition part that acquires electrocardiogram data indicating an electrocardiogram occurring in everyday life of a patient who is given the drug with the administration method and the dosage; and an output part that outputs information about advisability of modifying at least one of the administration method or the dosage on the basis of a result of determining, on the basis of the electrocardiogram data, whether a waveform abnormality that can be evaluated with an electrocardiogram may have occurred in the patient.

A system according to a fourth aspect of the present disclosure is a system used for determining at least one of an administration method or a dosage of a drug that can be administered to a patient, the system including: an information output apparatus; and an electrocardiograph, wherein the electrocardiograph includes: a measurement part that measures an electrocardiogram occurring in everyday life of the patient who is given the drug with the administration method and the dosage; and a transmitting part that transmits electrocardiogram data indicating the electrocardiogram; and the information output apparatus includes: an acquisition part that acquires the electrocardiogram data transmitted by the electrocardiograph; and an output part that outputs information about advisability of modifying at least one of the administration method or the dosage on the basis of a result of determining, on the basis of the electrocardiogram data, whether a waveform abnormality that can be evaluated with the electrocardiogram may have occurred in the patient.

### EFFECT OF THE INVENTION

According to the present disclosure, an effect that a medical worker can specify at least one of an administration method or a dosage of drugs more suitable for a patient is achieved.

### BRIEF DESCRIPTION OF THE DRAWINGS

FIG. 1 illustrates an outline of an information output system according to an embodiment.
FIG. 2 is a block diagram of the information output system according to the embodiment.
FIG. 3A is a schematic diagram of a normal electrocardiogram of a patient, and FIG. 3B is a schematic diagram of an initial dosage electrocardiogram of the patient.
FIGS. 4A and 4B are each a front view of a doctor's device displaying a support information screen.
FIG. 5 is a schematic diagram of a modified dosage electrocardiogram of the patient.
FIG. 6 is a flowchart of an information output method executed by the information output system according to the embodiment.

### DETAILED DESCRIPTION OF THE INVENTION

### [Outline of an information output system S]

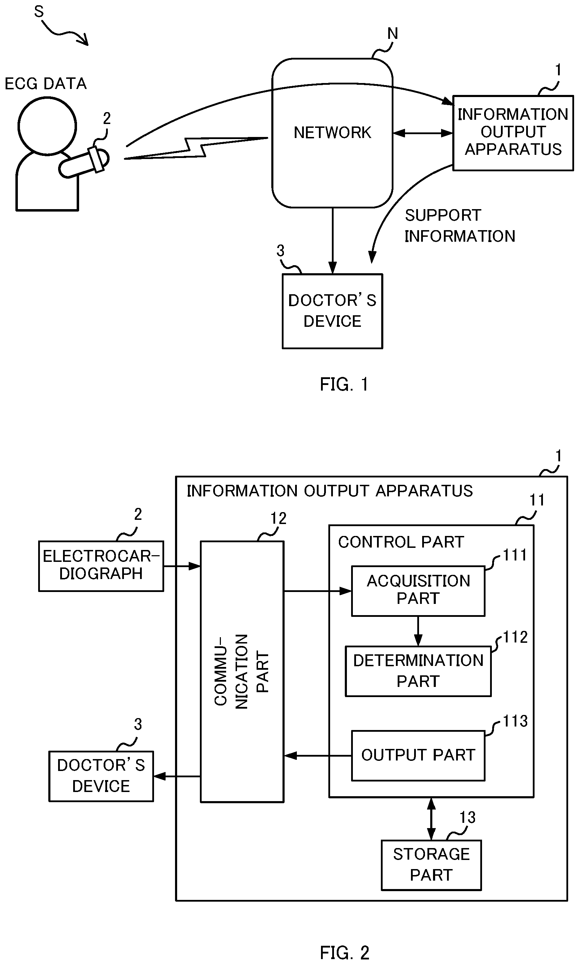

FIG. 1 illustrates an outline of an information output system S according to an embodiment. The information output system S is a system for supporting determination of at least one of an administration method or a dosage of a drug which may cause side effects that are detectable on the basis of an electrocardiogram. The information output system S is mainly applied to drugs whose administration methods and dosages are limited by side effects that are detectable on the basis of the electrocardiogram (for example, drugs where arrhythmia is a bottleneck). The information output system S includes an information output apparatus 1, an electrocardiograph 2, and a doctor's device 3.

The electrocardiograph 2 is an electrocardiograph worn by a patient, and is electrocardiogram measurement equipment that generates electrocardiogram data which indicates an electrocardiogram of the patient by measuring a pulse or a potential while being worn on his/her wrist, palm, chest, or the like, for example. The electrocardiograph 2 is a wearable electrocardiograph (continuously mounted electrocardiograph). The electrocardiograph 2 transmits the generated electrocardiogram data to the information output apparatus 1 via a network N including the wireless communication line. The electrocardiograph data generated by the electrocardiograph 2 may be sent to the information output apparatus 1 using a storage medium without passing through the network N, for example.

The doctor's device 3 is an information terminal used by a medical worker such as a doctor examining a patient, and includes a display and a computer, for example. The doctor's device 3 is associated in advance with a medical worker who uses the doctor's device 3 by an ID or the like given to the medical worker. The doctor's device 3 displays support information output by the information output apparatus 1 on the basis of the electrocardiogram data generated by the electrocardiograph 2.

The information output apparatus 1 is an apparatus that generates the support information for supporting determination of at least one of an administration method or a dosage of a drug on the basis of the electrocardiogram data generated by the electrocardiograph 2, and is a server, for example. The support information is information about the advisability of modifying at least one of the administration method or the dosage of the drug. The support information includes the electrocardiogram data and information indicating whether there is a possibility of occurrence of waveform abnormality in the electrocardiogram, for example.

FIG. 2 is a block diagram of the information output system S according to the embodiment. In FIG. 2, arrows indicate main data flows, and there may be data flows not shown in FIG. 2. In FIG. 2, each block indicates a configuration of a function unit, not a configuration of a hardware (device) unit. As such, the blocks shown in FIG. 2 may be implemented in a single device or separately in a plurality of devices. The transfer of data between the blocks may be performed via any means, such as a data bus, a network, a portable storage medium, or the like.

The information output apparatus 1 includes a control part 11, a communication part 12, and a storage part 13. The control part 11 includes an acquisition part 111, a determination part 112, and an output part 113. The communication part 12 has a communication controller for transmitting and receiving data between the electrocardiograph 2 and the doctor's device 3 via the network N. The communication part 12 notifies the control part 11 of the data received from the electrocardiograph 2 via the network N. Also, the communication part 12 transmits the data output from the control part 11 to the doctor's device 3 via the network N.

The storage part 13 is a storage medium including a read only memory (ROM), a random access memory (RAM), a hard disk, and the like. The storage part 13 stores in advance a program executed by the control part 11. The storage part 13 may be provided outside the information output apparatus 1, and in such a case, the storage part may exchange the data with the control part 11 via the network N.

The control part 11 is a processor such as a central processing unit (CPU), and functions as the acquisition part 111, the determination part 112, and the output part 113 by executing the program stored in the storage part 13, for example. At least some of the functions of the control part 11 may be performed by an electric circuit. Further, at least some of the functions of the control part 11 may be executed by the control part 11 executing a program executed via a network.

The information output system S according to the present embodiment is not limited to the specific configuration shown in FIG. 2. For example, the information output apparatus 1 may be formed by a single computer, may be formed by a plurality of computers cooperating with each other, or may be formed by a cloud which is a collection of computer resources. Two or more of the information output apparatus 1, the electrocardiograph 2, and the doctor's device 3 may be formed as one device.

### [Description of an information output method]

Hereinafter, an information output method executed by the information output system S according to the present embodiment will be described in detail. First, the electrocardiograph 2 measures an electrocardiogram of a patient in normal times. The patient in normal times is a patient in a state where a drug, for which the administration method and dosage are determined using the information output system S, has not been administered. The electrocardiograph 2 generates electrocardiogram data indicating the measured electrocardiogram and transmits the generated electrocardiogram data to the information output apparatus 1.

In the information output apparatus 1, the acquisition part 111 acquires, via the network N, the electrocardiogram data that is generated by the electrocardiograph 2 and indicates the electrocardiogram of the patient in normal times. The acquisition part 111 may acquire electrocardiogram data indicating the electrocardiogram of the patient in normal times measured in advance in a hospital or the like.

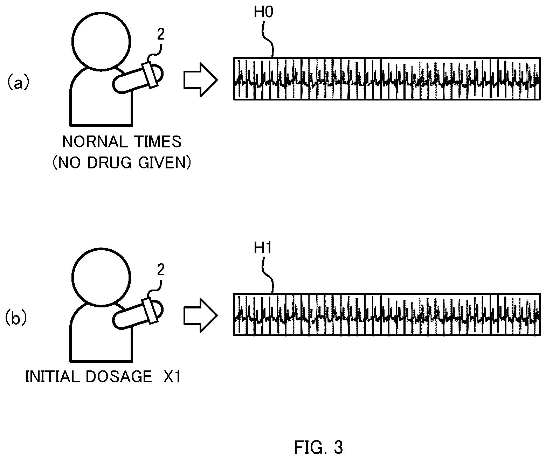

FIG. 3A is a schematic diagram of a normal electrocardiogram H0 of the patient in normal times. The determination part 112 determines, on the basis of the normal electrocardiogram H0 indicated by the electrocardiogram data acquired by the acquisition part 111, whether there is a possibility of occurrence of waveform abnormality that can be evaluated by the electrocardiogram. A method with which the determination part 112 determines, on the basis of the electrocardiogram, whether there is a possibility of occurrence of the waveform abnormality will be described later.

When the determination part 112 determines that there is a possibility of occurrence of the waveform abnormality in the patient in normal times, the output part 113 transmits, to the doctor's device 3, information indicating that the administration method and dosage cannot be determined on the basis of the waveform abnormality for said patient, and ends the process.

When the determination part 112 determines that there is no possibility of occurrence of the waveform abnormality in the patient in normal times, the output part 113 stores the electrocardiogram data of the normal electrocardiogram H0 of said patient in the storage part 13. The output part 113 does not need to store the electrocardiogram data of the normal electrocardiogram H0 in the storage part 13.

Next, the electrocardiograph 2 measures an electrocardiogram occurring in the everyday life of a patient who is given a drug at an initial dosage X1 for therapeutic purposes. The electrocardiograph 2 measures the electrocardiogram of the patient leading everyday life while the electrocardiograph 2 is worn on him/her. The patient living daily life may be active at home, at work, or the like, or may be hospitalized. The initial dosage X1 is a predetermined dosage (i.e., a dosage before a modification). The electrocardiograph 2 generates electrocardiogram data indicating the measured electrocardiogram and transmits the generated electrocardiogram data to the information output apparatus 1.

In the information output apparatus 1, the acquisition part 111 acquires, via the network N, electrocardiogram data which is generated by the electrocardiograph 2 and indicates an electrocardiogram of the patient who is administered the initial dosage X1. The acquisition part 111 may sequentially acquire the electrocardiogram data from the electrocardiograph 2 worn on the patient living daily life. By doing this, the information output apparatus 1 can detect a waveform abnormality on the electrocardiogram early which may occur in the patient on medication, and thus can enhance safety. Alternatively, the acquisition part 111 may collectively acquire the electrocardiogram data for a predetermined period from the electrocardiograph 2.

FIG. 3B is a schematic diagram of an initial dosage electrocardiogram H1 of the patient who is administered the initial dosage X1. The determination part 112 determines, on the basis of the initial dosage electrocardiogram H1 indicated by the electrocardiogram data acquired by the acquisition part 111, whether there is a possibility of occurrence of a waveform abnormality that can be evaluated by the electrocardiogram.

For example, the determination part 112 determines, as the waveform abnormality, whether there is a possibility of occurrence of a waveform indicating that arrhythmia is present or may occur in the electrocardiogram. By detecting at least one of QT prolongation, atrial fibrillation, ventricular tachycardia, supraventricular extrasystole, premature ventricular contraction, atrial flutter, or atrial tachycardia in an electrocardiogram, the determination part 112 determines whether there is the possibility of occurrence of a waveform indicating that arrhythmia is present or may occur, for example. That is, when a predetermined one or more of QT prolongation, atrial fibrillation, ventricular tachycardia, supraventricular extrasystole, premature ventricular contraction, atrial flutter, and atrial tachycardia are detected in response to the drug being administered to the patient, the determination part 112 determines that there is the possibility of occurrence of a waveform indicating that arrhythmia is present or may occur, and otherwise determines that there is no such possibility.

Specifically, the determination part 112 detects that a waveform predefined for each of QT prolongation, atrial fibrillation, ventricular tachycardia, supraventricular extrasystole, premature ventricular contraction, atrial flutter, and atrial tachycardia is appearing in the electrocardiogram. The determination part 112 detects QT prolongation when a waveform indicating that a time period from the beginning of the QRS wave to the end of the T wave is extended to a predetermined value or more appears in the electrocardiogram, for example. The determination part 112 detects atrial fibrillation when a waveform indicating that irregular, fine waves (f waves) are occurring appears in the electrocardiogram, for example. The determination part 112 detects ventricular tachycardia when a waveform indicating that the heart rate is equal to or greater than a predetermined value and that a width of the QRS wave is equal to or greater than a predetermined value appears in the electrocardiogram, for example. Similarly, the determination part 112 detects premature ventricular contraction, atrial flutter, or atrial tachycardia when the predetermined waveform appears in the electrocardiogram.

Alternatively, the determination part 112 may determine, as the waveform abnormality, whether there is a possibility of occurrence of a waveform indicating that an adverse event that can be evaluated by the electrocardiogram, and not limited to arrhythmia, is present. Not being in sinus rhythm is an example of the adverse event. In this case, when a waveform having a shape or a timing different from that of a normal sinus rhythm is detected in the electrocardiogram, the determination part 112 determines that there is a possibility of occurrence of a waveform indicating that the adverse event is present, and otherwise determines that there is no such possibility.

Also, the determination part 112 may determine whether there is a possibility of occurrence of a waveform abnormality by comparing the normal electrocardiogram H0 and the initial dosage electrocardiogram H1. In this case, the determination part 112 compares the normal electrocardiogram H0 indicated by the electrocardiogram data stored in the storage part 13 and the initial dosage electrocardiogram H1 indicated by the electrocardiogram data acquired by the acquisition part 111. If a difference (for example, a difference in QT time) between the normal electrocardiogram H0 and the initial dosage electrocardiogram H1 is equal to or greater than a predetermined value, the determination part 112 determines that there is a possibility of occurrence of a waveform abnormality, and otherwise, determines that there is no such possibility. By doing this, even if QT prolongation, atrial fibrillation, or ventricular tachycardia itself is not detected in the electrocardiogram, the determination part 112 can determine whether there is the possibility of occurrence of a waveform abnormality on the basis of a difference from normal times.

The determination part 112 is not limited to the determination method described herein, and may determine whether there is a possibility of occurrence of a waveform abnormality that can be evaluated by the electrocardiogram using other determination methods. Also, the determination part 112 may determine that the electrocardiogram is in sinus rhythm regardless of whether there is the possibility of occurrence of a waveform abnormality.

The determination part 112 may determine whether there is the possibility of occurrence of a waveform abnormality in the electrocardiogram using a machine learning model generated by learning electrocardiograms. In this case, the storage part 13 stores in advance a machine learning model generated by learning electrocardiograms of a normal state and an abnormal state using a known learning method. The electrocardiogram of the abnormal state is an electrocardiogram of a patient in which the predetermined one or more of the above-mentioned QT prolongation, atrial fibrillation, ventricular tachycardia, supraventricular extrasystole, premature ventricular contraction, atrial flutter, atrial tachycardia, and a sinus rhythm abnormality are occurring in response to the drug administered to him/her.

By inputting the electrocardiograms into the machine learning model stored in the storage part 13, the determination part 112 acquires a determination result indicating whether there is the possibility of occurrence of a waveform abnormality. In this way, even if a specific waveform corresponding to a waveform abnormality is not defined in advance, the determination part 112 can determine whether there is the possibility of occurrence of a waveform abnormality by using the machine learning model.

The output part 113 outputs, on the basis of the determination result of the determination part 112, support information about the advisability of modifying a dosage of the drug to the doctor's device 3 via the network N. The output part 113 outputs the support information including the electrocardiogram data and determination information indicating whether there is a possibility of occurrence of a waveform abnormality determined by the determination part 112, for example. When it is determined that a waveform abnormality determined by the determination part 112 is occurring, the determination information may indicate contents of the waveform abnormality that may have occurred.

Further, the output part 113 may specify an increase candidate value when the determination part 112 determines that there is no possibility of occurrence of a waveform abnormality. The increase candidate value is a value that is assumed to be used for increasing a dosage from the initial dosage X1, and is added to or multiplied by the initial dosage X1. The output part 113 specifies the increase candidate value in accordance with an increase table indicating increase candidate values stored in advance in the storage part 13, for example. The increase table may indicate fixed increase candidate values or may indicate increase candidate values that vary depending on the initial dosage X1. The output part 113 outputs support information including the specified increase candidate value in addition to the electrocardiogram data and the determination information.

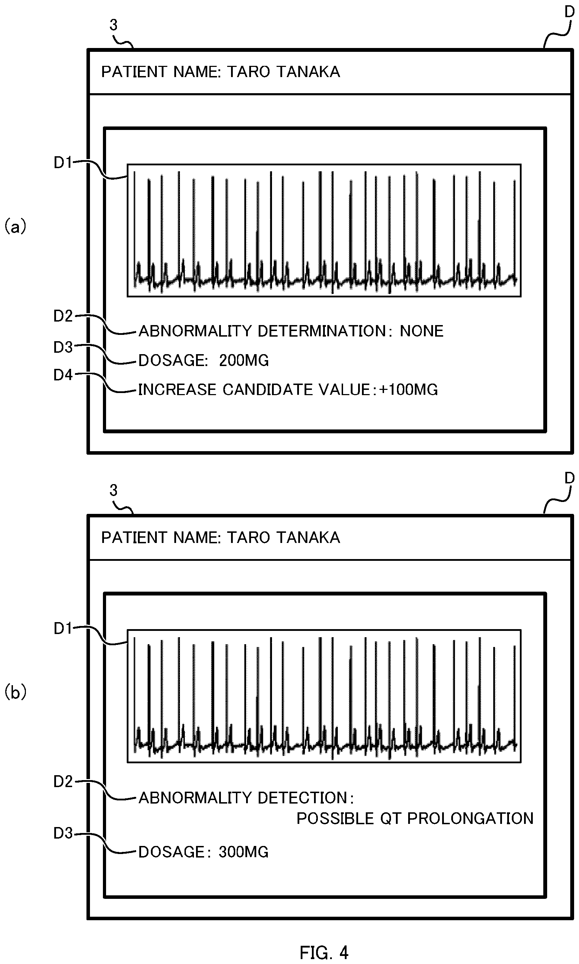

The doctor's device 3 displays a support information screen on a display (display part) on the basis of the support information output by the information output apparatus 1. FIGS. 4A and 4B are each a front view of the doctor's device 3 displaying a support information screen D.

FIG. 4A shows the support information screen D in a case where the determination part 112 of the information output apparatus 1 determines that there is no possibility of occurrence of a waveform abnormality. In this case, the support information screen D includes an electrocardiogram D1, determination information D2, a dosage D3, and an increase candidate value D4. The electrocardiogram D1 is an electrocardiogram indicated by the electrocardiogram data. The determination information D2 is information representing that there is no possibility of occurrence of a waveform abnormality. The dosage D3 is an amount (here, the initial dosage X1) of a drug administered to the patient. The increase candidate value D4 is an increase candidate value specified by the output part 113 of the information output apparatus 1.

FIG. 4B shows the support information screen D in a case where the determination part 112 of the information output apparatus 1 determines that there is a possibility of occurrence of a waveform abnormality. In this case, the support information screen D includes the electrocardiogram D1, the determination information D2, and the dosage D3. The electrocardiogram D1 may represent the overall electrocardiogram indicated by the electrocardiogram data, or may represent an electrocardiogram of a portion, extracted from the electrocardiogram data, determined to be indicating the occurrence of the waveform abnormality. The determination information D2 is information representing the contents of the waveform abnormality. The dosage D3 is an amount of a drug administered to the patient.

As described above, by referencing the support information displayed on the support information screen D, a medical worker can easily grasp whether the waveform abnormality that can be evaluated by the electrocardiogram has occurred in the patient on medication, and specify a more suitable dosage of the drug for said patient.

The medical worker determines the advisability of increasing the dosage on the basis of the support information, and determines a modified dosage X2 which is a dosage to be administered to the patient next. For example, the medical worker determines, as the modified dosage X2, a value obtained by (i) adding the increase candidate value D4 to the dosage D3 or (ii) multiplying the dosage D3 by the increase candidate value D4. Further, the medical worker may determine other values as the modified dosage X2 on the basis of the support information. The medical worker may input the determined modified dosage X2 by operating the doctor's device 3.

After the dosage is modified, i.e. after the modified dosage X2 is determined, the electrocardiograph 2 measures an electrocardiogram occurring in everyday life of the patient who is given the drug at the modified dosage X2. The electrocardiograph 2 generates electrocardiogram data representing the measured electrocardiogram, and transmits the electrocardiogram data to the information output apparatus 1. The acquisition part 111 re-acquires, via the network N, the electrocardiogram data generated by the electrocardiograph 2 and indicating the electrocardiogram of the patient who is administered the modified dosage X2.

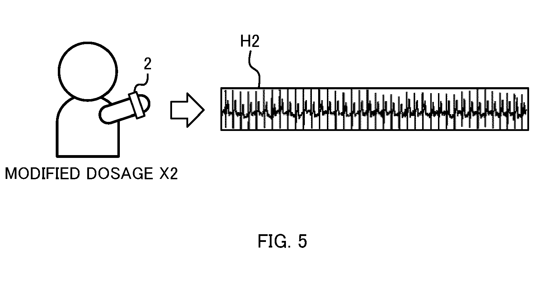

FIG. 5 is a schematic diagram of a modified dosage electrocardiogram H2 of a patient who is administered a modified dosage X2. The determination part 112 determines whether there is a possibility of occurrence of a waveform abnormality that can be evaluated by the electrocardiogram, on the basis of the modified dosage electrocardiogram H2 indicated by the electrocardiogram data re-acquired by the acquisition part 111. The method by which the determination part 112 determines whether there is a possibility of occurrence of the waveform abnormality on the basis of the modified dosage electrocardiogram H2 is the same as in the case of the initial dosage electrocardiogram H1.

The output part 113 outputs, on the basis of the determination result of the determination part 112, the support information about the advisability of modifying the dosage of the drug to the doctor's device 3 via the network N. The output part 113 re-outputs the support information including the electrocardiogram data and the determination information indicating whether there is a possibility of occurrence of a waveform abnormality determined by the determination part 112, for example. When it is determined that there is a possibility of occurrence of a waveform abnormality determined by the determination part 112, the determination information may indicate the contents of the waveform abnormality that may have occurred.

Further, the output part 113 may specify an increase candidate value when the determination part 112 determines that there is no possibility of occurrence of a waveform abnormality. The increase candidate value is a value that is assumed to be used for increasing a dosage from the modified dosage X2, and is added to or multiplied by the modified dosage X2. The method by which the output part 113 specifies the increase candidate value is the same as in the case of the initial dosage X1. The output part 113 re-outputs the support information including the specified increase candidate value in addition to the electrocardiogram data and the determination information.

Further, the output part 113 may output support information including risk information representing the degree of risk of increasing the dosage. In this case, the output part 113 compares a plurality of electrocardiograms corresponding to a plurality of dosages (e.g., the initial dosage X1 and the modified dosage X2), and specifies the degree of risk such that the greater a difference (e.g., the difference in QT time) between them, the greater the degree of risk, and the smaller the difference, the smaller the degree of risk. The output part 113 outputs the risk information representing the specified degree of risk in addition to the electrocardiogram data and the determination information. In this way the medical worker can grasp not only the presence or absence of the waveform abnormality but also the degree of risk of increasing the dosage by referencing the support information, and thus can easily determine the advisability of increasing the dosage.

The doctor's device 3 displays the support information screen shown in FIGS. 4A and 4B again on the display on the basis of the support information output by the information output apparatus 1. The medical worker determines the advisability of increasing the dosage on the basis of the support information again, and determines the modified dosage X2 to be administered to the patient next.

The information output system S repeats acquiring of the electrocardiogram of the patient who is administered the modified dosage X2 and outputting of the support information, until the modified dosage X2 reaches a predetermined upper limit value or until the increase reaches its predetermined number of times. When the modified dosage X2 reaches the predetermined upper limit value or when the increase reaches its predetermined number of times, the medical worker determines, as the dosage suitable for the patient, the maximum initial dosage X1 or the maximum modified dosage X2 among the initial dosages X1 or the modified dosages X2 used when the determination part 112 determines that there is no possibility of occurrence of a waveform abnormality, for example. In this way, the medical worker can efficiently find, on the basis of the support information, a dosage of the drug more suitable for the patient by repeatedly trying out increasing the dosage within a range in which no waveform abnormalities occur.

When the determination part 112 determines that there is a possibility of occurrence of a waveform abnormality in the modified dosage electrocardiogram H2 of the patient who is administered the modified dosage X2, the medical worker may reduce the modified dosage X2 on the basis of the support information. For example, the medical worker determines, as the reduced modified dosage X2, a value obtained by (i) subtracting a predetermined value from the modified dosage X2 or (ii) dividing the modified dosage X2 by the predetermined value.

The electrocardiograph 2 measures an electrocardiogram occurring in everyday life of a patient who is given the drug at the reduced modified dosage X2. The electrocardiograph 2 generates electrocardiogram data representing the measured electrocardiogram, and transmits the electrocardiogram data to the information output apparatus 1. The information output apparatus 1 determines whether there is a possibility of occurrence of a waveform abnormality in the modified dosage electrocardiogram H2 of the patient who is administered the reduced modified dosage X2, and re-outputs the support information. The doctor's device 3 displays the support information screen shown in FIGS. 4A and 4B on the display on the basis of the support information output by the information output apparatus 1 again.

When the determination part 112 determines that there is no possibility of occurrence of a waveform abnormality in the reduced modified dosage X2, the medical worker determines the reduced modified dosage X2 as the dosage suitable for the patient. The medical worker may further reduce the modified dosage X2 or may stop modifying the dosage when the determination part 112 determines that there is a possibility of occurrence of a waveform abnormality in the reduced modified dosage X2. In this way, the medical worker can efficiently find a dosage of the drug more suitable for the patient not only by increasing but also by decreasing the dosage.

By modifying not only the dosage of the drug but also the administration method of the drug, the information output apparatus 1 may make the administration method of the drug more suitable for the patient specifiable by repeating the acquiring of the electrocardiogram of the patient and the outputting of the support information. In this case, in the information output apparatus 1, the acquisition part 111 acquires, via the network N, electrocardiogram data generated by the electrocardiograph 2 and indicating the electrocardiogram of the patient who is administered the predetermined dosage using a certain administration method (for example, administered three times a day). The determination part 112 determines, on the basis of the electrocardiogram of the predetermined dosage and administration method, whether there is a possibility of occurrence of a waveform abnormality, using the above-described method.

The output part 113 outputs the support information to the doctor's device 3 via the network N on the basis of the determination result of the determination part 112. The medical worker determines, on the basis of the support information output by the information output apparatus 1, the advisability of modifying the administration method, and determines the administration method to be used for the patient next. In this case, the medical worker may modify (i) only the administration method or (ii) both the administration method and the dosage. For example, the medical worker modifies the administration method of administering three times a day to the administration method of administering four times a day. Similar to the case where only the dosage is modified, the medical worker repeatedly tries out, on the basis of the support information, the modification of at least one of the administration method or the dosage within the range in which no waveform abnormalities occur, and determines at least one of the administration method or the dosage suitable for the patient.

As described above, by referencing the support information output by the information output apparatus 1, the medical worker can easily grasp whether a waveform abnormality that can be evaluated by the electrocardiogram is occurring in the patient on medication while repeatedly trying out the modification of at least one of the administration method or the dosage within the range in which no waveform abnormalities occur, and specify at least one of an administration method or a dosage of the drug more suitable for the patient.

### [Modified example]

In the above embodiments, the case where the information output system S is used to determine at least one of the administration method or the dosage when the information output system S administers medication to an actual patient is exemplified, but the information output system S may also be used to determine at least one of the administration method or the dosage in clinical trials of drugs. In this case, the electrocardiograph 2 is attached to a subject participant, serving as a patient, to whom medication is administered for the purpose of clinical trials. The electrocardiograph 2 measures an electrocardiogram of the subject participant who is given a drug with a predetermined administration method and dosage. The information output apparatus 1 determines whether there is a possibility of occurrence of a waveform abnormality in the subject participant to whom the drug is administered with the predetermined dosage and administration method, and outputs the support information.

The medical worker references the support information output by the information output apparatus 1 on the basis of the electrocardiogram of the subject participant while repeatedly trying out the modification of at least one of the administration method or the dosage within the range in which no waveform abnormalities occur. The medical worker determines, as the administration method and dosage of the drug, one of an administration method and a dosage among the administration methods and the dosages used when it is determined that there is no possibility of occurrence of a waveform abnormality, on the basis of the support information. In this way, the medical worker can efficiently find, on the basis of the support information, at least one of an appropriate administration method or an appropriate dosage of the drug, within a range in which no waveform abnormality occurs in the subject participant.

### [Flow of the information output method]

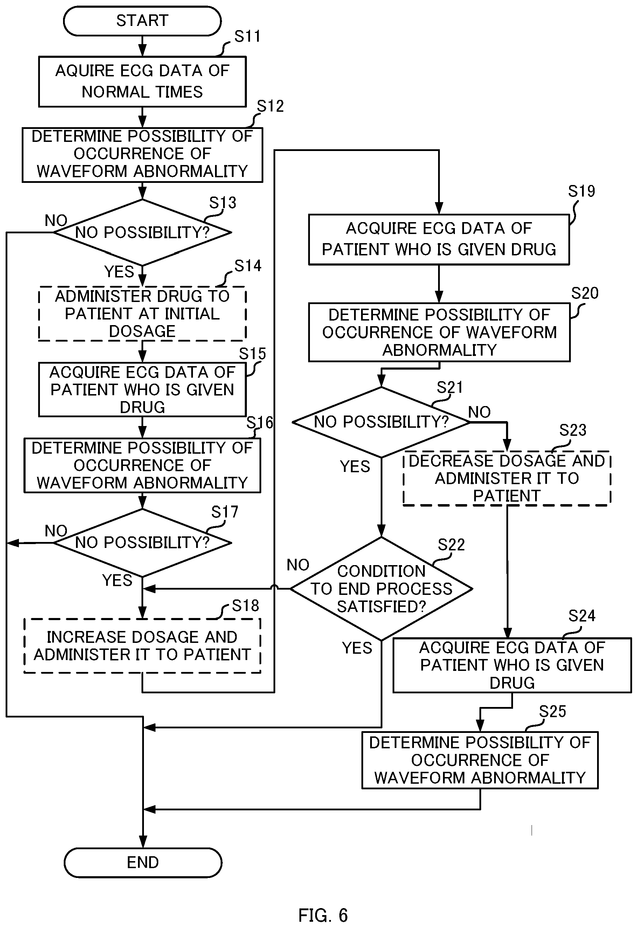

FIG. 6 is a flowchart of the information output method executed by the information output system S according to the embodiment. Although the flowchart illustrated in FIG. 6 shows a case where the dosage of the drug is modified as an example, the same applies to a case where the administration method of the drug is modified. In FIG. 6, steps not performed by the information output system S (specifically, steps of administering medication to a patient) are represented by broken lines.

First, the electrocardiograph 2 measures an electrocardiogram of a patient in normal times. The electrocardiograph 2 generates electrocardiogram data indicating the measured electrocardiogram and transmits the generated electrocardiogram data to the information output apparatus 1. In the information output apparatus 1, the acquisition part 111 acquires, via the network N, the electrocardiogram data that is generated by the electrocardiograph 2 and indicates the electrocardiogram of the patient in normal times (S11).

The determination part 112 determines, on the basis of a normal electrocardiogram indicated by the electrocardiogram data acquired by the acquisition part 111, whether there is a possibility of occurrence of a waveform abnormality that can be evaluated by the electrocardiogram (S12). When the determination part 112 determines that there is a possibility of occurrence of a waveform abnormality in the patient in normal times (NO in S13), the output part 113 transmits, to the doctor's device 3, information indicating that the administration method and dosage cannot be determined on the basis of the waveform abnormality for said patient, and ends the process.

When the determination part 112 determines that there is no possibility of occurrence of a waveform abnormality in the patient in normal times (YES in S13), the output part 113 stores the electrocardiogram data of the normal electrocardiogram of said patient in the storage part 13.

The medical worker or the patient administers a drug to the patient at the initial dosage X1 (S14). The electrocardiograph 2 measures an electrocardiogram occurring in everyday life of the patient who is given the drug at the initial dosage X1. The electrocardiograph 2 generates electrocardiogram data indicating the measured electrocardiogram and transmits the electrocardiogram data to the information output apparatus 1. In the information output apparatus 1, the acquisition part 111 acquires, via the network N, electrocardiogram data which is generated by the electrocardiograph 2 and indicates an electrocardiogram of the patient who is administered the initial dosage X1 (S15).

The determination part 112 determines, on the basis of an initial dosage electrocardiogram indicated by the electrocardiogram data acquired by the acquisition part 111, whether there is a possibility of occurrence of a waveform abnormality that can be evaluated by the electrocardiogram (S16). When the determination part 112 determines that there is a possibility of occurrence of a waveform abnormality in the patient who is administered the initial dosage X1 (NO in S17), the output part 113 transmits support information including the electrocardiogram data and determination information to the doctor's device 3. The doctor's device 3 displays the support information output by the information output apparatus 1 and ends the process.

When the determination part 112 determines that there is no possibility of occurrence of a waveform abnormality in the patient who is administered the initial dosage X1 (YES in S17), the output part 113 transmits the support information including the electrocardiogram data, the determination information, and an increase candidate value to the doctor's device 3. The doctor's device 3 displays the support information output by the information output apparatus 1.

The medical worker determines the advisability of increasing the dosage on the basis of the support information, and determines a modified dosage X2 to be administered to the patient next. The medical worker or the patient administers the drug to the patient at the modified dosage X2 (S18).

The electrocardiograph 2 measures an electrocardiogram occurring in everyday life of a patient who is given the drug at the modified dosage X2. The electrocardiograph 2 generates electrocardiogram data indicating the measured electrocardiogram, and transmits the electrocardiogram data to the information output apparatus 1. The acquisition part 111 re-acquires, via the network N, electrocardiogram data generated by the electrocardiograph 2 and indicating the electrocardiogram of the patient who is administered the modified dosage X2 (S19).

The determination part 112 determines whether there is a possibility of occurrence of a waveform abnormality that can be evaluated by the electrocardiogram on the basis of the modified dosage electrocardiogram indicated by the electrocardiogram data re-acquired by the acquisition part 111 (S20). When the determination part 112 determines that there is no possibility of occurrence of a waveform abnormality in the patient who is administered the modified dosage X2 (YES in S21) and a predetermined condition to end the process (for example, the modified dosage X2 reached the upper limit value) is not satisfied (NO in S22), the output part 113 transmits the support information including the electrocardiogram data, the determination information, and the increase candidate value to the doctor's device 3.

Here, the output part 113 may output support information including risk information representing a degree of risk of increasing the dosage on the basis of (i) the initial administration method electrocardiogram indicated by the electrocardiogram data of the patient who is administered the initial dosage X1 and (ii) the modified administration method electrocardiogram indicated by the electrocardiogram data of the patient who is administered the modified dosage X2.

The doctor's device 3 displays the support information output by the information output apparatus 1. The medical worker determines the advisability of increasing the dosage on the basis of the support information again, and determines the modified dosage X2 to be administered to the patient next. The information output system S repeats Steps S18 to S21.

When the determination part 112 determines that there is no possibility of occurrence of a waveform abnormality in the patient who is administered the modified dosage X2 (YES in S21) or when the predetermined condition to end the process is satisfied (YES in S22), the output part 113 transmits the support information including the electrocardiogram data and the determination information to the doctor's device 3. The doctor's device 3 displays the support information output by the information output apparatus 1. For example, the medical worker determines, as the dosage suitable for the patient, the maximum initial dosage X1 or the maximum modified dosage X2 among the initial dosages X1 or the modified dosages X2 used when the determination part 112 determines that there is no possibility of occurrence of a waveform abnormality,.

When the determination part 112 determines that there is a possibility of occurrence of a waveform abnormality in the patient who is administered the modified dosage X2 (NO in S21), the output part 113 transmits the support information including the electrocardiogram data and the determination information to the doctor's device 3. The doctor's device 3 displays the support information output by the information output apparatus 1. The medical worker determines the reduced modified dosage X2 on the basis of the support information. The medical worker or patient administers the drug to the patient at the reduced modified dosage X2 (S23). The information output system S repeats Steps S19 to S21.

The electrocardiograph 2 measures an electrocardiogram occurring in everyday life of the patient who is given the drug at the reduced modified dosage X2. The electrocardiograph 2 generates electrocardiogram data indicating the measured electrocardiogram, and transmits the electrocardiogram data to the information output apparatus 1. The acquisition part 111 acquires, via the network N, the electrocardiogram data generated by the electrocardiograph 2 and indicating the electrocardiogram of the patient who is administered the reduced modified dosage X2 (S24).

The determination part 112 determines whether there is a possibility of occurrence of a waveform abnormality that can be evaluated by the electrocardiogram on the basis of the modified administration method electrocardiogram indicated by the electrocardiogram data acquired by the acquisition part 111 (S25). The output part 113 transmits the support information including the electrocardiogram data and the determination information to the doctor's device 3. The doctor's device 3 displays the support information output by the information output apparatus 1. When the determination part 112 determines that there is no possibility of occurrence of a waveform abnormality in the reduced modified dosage X2, the medical worker determines the reduced modified dosage X2 as the dosage suitable for the patient. The medical worker may further reduce the modified dosage X2 or may stop modifying the dosage when the determination part 112 determines that there is a possibility of occurrence of a waveform abnormality in the reduced modified dosage X2.

### [Effect of embodiments]

According to the information output system S of the present embodiment, the information output apparatus 1 determines whether there is a possibility of occurrence of a waveform abnormality on the basis of the electrocardiogram occurring in everyday life of the patient on medication, and transmits the support information about the advisability of modifying at least one of the administration method or the dosage of the drug to the doctor's device 3. By referencing the support information in the doctor's device 3, the medical worker can easily grasp whether a waveform abnormality that can be evaluated by the electrocardiogram has occurred in the patient on medication while repeatedly trying out the modification of at least one of the administration method or the dosage within the range in which no waveform abnormalities occur, and can specify at least one of an administration method or a dosage more suitable for the patient.

The present disclosure is explained based on the exemplary embodiments. The technical scope of the present disclosure is not limited to the scope explained in the above embodiments and it is possible to make various changes and modifications within the scope of the disclosure. For example, all or part of the apparatus can be configured with any unit which is functionally or physically dispersed or integrated. Further, new exemplary embodiments generated by arbitrary combinations of them are included in the exemplary embodiments. Further, effects of the new exemplary embodiments brought by the combinations also have the effects of the original exemplary embodiments.

The processor of the information output apparatus 1 performs each step (process) included in the information output method shown in FIG. 6. That is, the processor of the information output apparatus 1 reads, from the storage part, a program for executing the information output method shown in FIG. 6, and executes the program to control each unit of the information output system S, thereby executing the information output method shown in FIG. 6. The steps included in the information output method shown in FIG. 6 may be partially omitted, the order between the steps may be changed, or a plurality of steps may be performed in parallel.

### [Description of the reference numerals]

- S: Information output system
- 1: Information output apparatus
- 11: Control part
- 111: Acquisition part
- 112: Determination part
- 113: Output part
- 2: Electrocardiograph
- 3: Doctor's device

## Claims

1. A method used for determining at least one of an administration method or a dosage of a drug that can be administered to a patient, the method executed by a processor and including steps of:
acquiring electrocardiogram data indicating an electrocardiogram occurring in everyday life of a patient who is given the drug with the administration method and the dosage;
determining, on the basis of the electrocardiogram data, whether a waveform abnormality that can be evaluated with an electrocardiogram may have occurred in the patient; and
outputting information about advisability of modifying at least one of the administration method or the dosage on the basis of a result of the determining.

2. The method according to claim 1, further comprising steps of:
re-acquiring, after at least one of the administration method or the dosage has been modified, electrocardiogram data of the patient who is given the drug with the modified administration method and dosage;
determining whether there is the possibility, on the basis of the re-acquired electrocardiogram data; and
re-outputting information about the advisability of modifying at least one of the administration method or the dosage on the basis of a result of the determining.

3. The method according to claim 1 or 2, wherein the acquiring step sequentially acquires the electrocardiogram data from an electrocardiograph worn by the patient in his/her daily life.

4. The method according to any one of claims 1 to 3, wherein the outputting step determines, as the waveform abnormality, whether there is a possibility of occurrence of a waveform indicating that arrhythmia is present or may occur.

5. The method according to any one of claims 1 to 4, wherein the outputting step determines, as the waveform abnormality, whether there is a possibility of occurrence of a waveform indicating that an adverse event that can be evaluated with an electrocardiogram is present.

6. The method according to any one of claims 1 to 5, wherein the outputting step determines whether there is a possibility of occurrence of the waveform abnormality by detecting at least one of QT prolongation, atrial fibrillation, ventricular tachycardia, supraventricular extrasystole, premature ventricular contraction, atrial flutter, or atrial tachycardia in the electrocardiogram data.

7. The method according to any one of claims 1 to 6, wherein the outputting step determines whether there is a possibility of occurrence of the waveform abnormality by comparing the electrocardiogram data and normal electrocardiogram data indicating an electrocardiogram of the patient in normal times.

8. The method according to any one of claims 1 to 7, wherein the outputting step transmits information about the advisability of modifying at least one of the administration method or the dosage to an information terminal associated with a medical worker who examines the patient.

9. The method according to claim 2, wherein the re-outputting step outputs information representing a risk of increasing the dosage on the basis of (i) the electrocardiogram data of the patient who is given the drug at the dosage and (ii) the electrocardiogram data of the patient who is given the drug at the modified dosage.

10. The method according to any one of claims 1 to 9, wherein the obtaining step obtains the electrocardiogram data of the patient who is given the drug for therapeutic purposes or for purposes of clinical trials.

11. A program used for determining at least one of an administration method or a dosage of a drug that can be administered to a patient, the program causing a processor to execute steps of:
acquiring electrocardiogram data indicating an electrocardiogram occurring in everyday life of a patient who is given the drug with the administration method and the dosage;
determining, on the basis of the electrocardiogram data, whether a waveform abnormality that can be evaluated with an electrocardiogram may have occurred in the patient; and
outputting information about advisability of modifying at least one of the administration method or the dosage on the basis of a result of the determining.

12. An apparatus used for determining at least one of an administration method and a dosage of a drug that can be administered to a patient, the apparatus including:
an acquisition part that acquires electrocardiogram data indicating an electrocardiogram occurring in everyday life of a patient who is given the drug with the administration method and the dosage; and
an output part that outputs information about advisability of modifying at least one of the administration method or the dosage on the basis of a result of determining, on the basis of the electrocardiogram data, whether a waveform abnormality that can be evaluated with an electrocardiogram may have occurred in the patient.

13. A system used for determining at least one of an administration method or a dosage of a drug that can be administered to a patient, the system comprising:
an information output apparatus; and
an electrocardiograph, wherein
the electrocardiograph includes:
a measurement part that measures an electrocardiogram occurring in everyday life of the patient who is given the drug with the administration method and the dosage; and
a transmitting part that transmits electrocardiogram data indicating the electrocardiogram;
and
the information output apparatus includes:
an acquisition part that acquires the electrocardiogram data transmitted by the electrocardiograph; and
an output part that outputs information about advisability of modifying at least one of the administration method or the dosage on the basis of a result of determining, on the basis of the electrocardiogram data, whether a waveform abnormality that can be evaluated with the electrocardiogram may have occurred in the patient.
